(19) Europäisches Patentamt / European Patent Office / Office européen des brevets

(11) **EP 4 206 232 B1**

(12) **EUROPEAN PATENT SPECIFICATION**

(45) Date of publication and mention of the grant of the patent:
**19.02.2025 Bulletin 2025/08**

(21) Application number: **21902667.1**

(22) Date of filing: **08.12.2021**

(51) International Patent Classification (IPC):
***C08B 37/02*** *(2006.01)* ***C12P 19/08*** *(2006.01)*
***C12P 1/00*** *(2006.01)* ***C12N 1/20*** *(2006.01)*
***C12P 19/04*** *(2006.01)* ***A01N 43/16*** *(2006.01)*
***A01N 43/90*** *(2006.01)* ***A01P 21/00*** *(2006.01)*
***C12R 1/41*** *(2006.01)*

(52) Cooperative Patent Classification (CPC):
**A01P 21/00; A01N 43/16; A01N 43/90; C08B 37/0006; C08B 37/0021; C12N 1/20; C12N 9/2405; C12N 9/244; C12P 19/04; C12P 19/14; C12Y 302/01;** C12R 2001/01

(86) International application number:
**PCT/CN2021/136586**

(87) International publication number:
**WO 2022/121962 (16.06.2022 Gazette 2022/24)**

(54) **HETEROPOLY OLIGOSACCHARIDE AND APPLICATION THEREOF IN IMPROVING PLANT DISEASE RESISTANCE**

HETEROPOLYOLIGOSACCHARID UND ANWENDUNG DAVON ZUR VERBESSERUNG DER RESISTENZ GEGEN PFLANZENKRANKHEITEN

HÉTÉROPOLY-OLIGOSACCHARIDE ET SON APPLICATION DANS L'AMÉLIORATION DE LA RÉSISTANCE DE PLANTES AUX MALADIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 CN 202011427267**

(43) Date of publication of application:
**05.07.2023 Bulletin 2023/27**

(73) Proprietor: **CHANGZHOU ENDELAN CHEMICALS IMP. & EXP. CO., LTD.**
**Changzhou, Jiangsu 213022 (CN)**

(72) Inventors:
- **ZHANG, Jianfa**
  **Nanjing, Jiangsu 210094 (CN)**
- **FU, Renjie**
  **Nanjing, Jiangsu 210094 (CN)**
- **LI, Jing**
  **Nanjing, Jiangsu 210094 (CN)**
- **CHENG, Rui**
  **Nanjing, Jiangsu 210094 (CN)**

(74) Representative: **Sun, Yiming**
**HUASUN Patent- und Rechtsanwälte**
**Friedrichstraße 33**
**80801 München (DE)**

(56) References cited:
**EP-A1- 0 040 445** **CN-A- 105 566 510**
**CN-A- 111 286 466** **US-A- 5 125 099**

- **YANG YUNXIA ET AL: "Anti-tumor activity and immunogenicity of a succinoglycan riclin", CARBOHYDRATE POLYMERS, APPLIED SCIENCE PUBLISHERS , LTD BARKING, GB, vol. 255, 12 November 2020 (2020-11-12), XP086440893, ISSN: 0144-8617, [retrieved on 20201112], DOI: 10.1016/ J.CARBPOL.2020.117370**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHOULY ET AL: "NMR studies of succinoglycan repeating-unit octasaccharides from Rhizobium meliloti and Agrobacterium radiobacter", INTERNATIONAL JOURNAL OF BIOLOGICAL MACROMOLECULES, ELSEVIER BV, NL, vol. 17, no. 6, 1 January 1995 (1995-01-01), pages 357 - 363, XP005295703, ISSN: 0141-8130, DOI: 10.1016/0141-8130(96)81846-0**
- **CHENG R. ET AL: "In vitro and in vivo anti-inflammatory activity of a succinoglycan Riclin from Agrobacterium sp. ZCC3656", JOURNAL OF APPLIED MICROBIOLOGY, vol. 127, no. 6, 21 October 2019 (2019-10-21), GB, pages 1716 - 1726, XP093125170, ISSN: 1364-5072, Retrieved from the Internet <URL:https://onlinelibrary.wiley.com/doi/full-xml/10.1111/jam.14447> DOI: 10.1111/jam.14447**
- **FU RENJIE ET AL: "Transcriptomic and metabolomic profiling revealed the role of succinoglycan Riclin octaose in eliciting the defense response of Solanum tuberosum", APPLIED MICROBIOLOGY AND BIOTECHNOLOGY, SPRINGER BERLIN HEIDELBERG, BERLIN/HEIDELBERG, vol. 105, no. 19, 18 September 2021 (2021-09-18), pages 7439 - 7450, XP037582744, ISSN: 0175-7598, [retrieved on 20210918], DOI: 10.1007/S00253-021-11588-1**
- **WANG LEI, CHENG RUI, SUN XIAQING, ZHAO YANG, GE WENHAO, YANG YUNXIA, GAO YAN, DING ZHAO, LIU JUNHAO, ZHANG JIANFA: "Preparation and Gut Microbiota Modulatory Property of the Oligosaccharide Riclinoctaose", JOURNAL OF AGRICULTURAL AND FOOD CHEMISTRY, AMERICAN CHEMICAL SOCIETY, US, vol. 69, no. 12, 31 March 2021 (2021-03-31), US , pages 3667 - 3676, XP055940823, ISSN: 0021-8561, DOI: 10.1021/acs.jafc.0c07783**
- **ZHAO YANG, DING ZHAO, GE WENHAO, LIU JUNHAO, XU XI, CHENG RUI, ZHANG JIANFA: "Riclinoctaose Attenuates Renal Ischemia-Reperfusion Injury by the Regulation of Macrophage Polarization", FRONTIERS IN PHARMACOLOGY, vol. 12, 13 October 2021 (2021-10-13), XP055940826, DOI: 10.3389/fphar.2021.745425**
- **AMEMURA, A. ; MOORI, K. ; HARADA, T.: "Purification and properties of a specific, inducible @b-glucanase, succinoglucan depolymerase from Flavobacterium", BBA ENZYMOLOGY., ELSEVIER, AMSTERDAM., NL, vol. 334, no. 2, 21 February 1974 (1974-02-21), NL , pages 398 - 409, XP023967698, ISSN: 0005-2744, DOI: 10.1016/0005-2744(74)90183-1**
- **QU JUANJUAN: "Optimization of the Synthetic Process of Cyclic β-1,2-glucans by Rhizobium Radiobacter ATCC 1333", CHINESE MASTER'S THESES FULL-TEXT DATABASE, ENGINEERING SCIENCE VOL. I, no. 1, 15 January 2019 (2019-01-15), XP055940831**
- **YUE FEI-FEI, NI LI-XIAO;WANG YI-FEI;WANG NA;LIU XUAN-YU: "Study on the Enzymatic Hydrolysis of Extracellular Polysaccharide in Constructed Wetland Reactor by β-glucanase", ENVIRONMENTAL SCIENCE AND TECHNOLOGY, vol. 33, no. 2, 30 April 2020 (2020-04-30), XP055940840, ISSN: 1674-4829, DOI: 10.19824/j.cnki.cn32-1786/x.2020.0017**

## Description

### FIELD OF THE DISCLOSURE

The present invention relates to an application of a heteropoly oligosaccharide in improving disease resistance of a plant.

### BACKGROUND

Effective autoimmune systems have been formed in plants in response to invasions of pathogenic bacteria through long-term evolution and development. Plant resistance inducers can stimulate immune systems of plants to resist, prevent and treat diseases. With respect to interactions between plants and pathogens, on one hand, plants secrete β-1,3 glucanase, chitosanase and chitinase to directly hydrolyze cell walls of pathogenic bacteria and inhibit their growth; on the other hand, pathogenic bacteria secrete polygalacturonase and pectinase to degrade cell walls of plants. Oligosaccharide fragments produced through the mutual hydrolyses can stimulate plants to produce pathogenesis-related proteins, phytoalexins, etc., thereby enhancing plant disease resistance. This is the basic principle of oligosaccharides as plant disease resistance inducers.

At present, research on the mechanism of oligosaccharides has developed from general observation to molecular and cellular levels. A large number of studies reveal that oligosaccharides play a role of elicitors generally in the following manner. Mutual recognition of oligosaccharides and receptors on cell membranes causes changes in receptor conformation and produces transmembrane signals; through a series of signal transduction, amplification and integration, expression of defense genes is regulated, secondary metabolites are accumulated, and plants are induced to produce autoimmune resistance to infection of pathogenic substances.

Oligosaccharide elicitors are derived from natural products and have the following functional characteristics according to the current research. (1) They are ecologically-friendly. (2) They are effective against pathogenic bacteria that are ineffectively prevented and treated by conventional methods, especially those resistant to chemical pesticides. (3) They act on plants rather than directly on pathogenic organisms, which avoids adverse effects on non-pathogenic organisms. (4) They protect plants from various biotic stresses of microorganisms, insects, nematodes, etc. (5) They can be combined with other prevention and treatment methods acting in different ways to expand the scope of prevention and treatment. (6) They activate various genes for systemic resistance, some of which may protect plants from high and low temperatures, drought and ultraviolet stresses.

Oligosaccharide elicitors that have been extensively studied in recent years mainly include the following types. (1) Chitosan oligosaccharides: oligosaccharide products with a degree of polymerization (DP) between 2 and 20 obtained through the degradation of chitosan by means of a special biological enzyme technology; can induce plant disease resistance, elicit immunity against and kill various fungi, bacteria and viruses; have favorable prevention and treatment effects on cotton verticillium wilt, rice blast, tomato late blight and other diseases; and can be developed into biological pesticides, growth regulators, fertilizers, etc. (2) Chitin oligosaccharides: degradation products of chitin, are recognized as a general term of saccharides composed of 2-10 N-acetylglucosamines linked by glycosidic bonds, and have a good inhibitory effect on various pathogenic bacteria in plants, such as those causing wheat sharp eyespot and tobacco black shank. (3) Glucooligosaccharides: a series of oligosaccharide elicitors that people studied at first and knew systematically; can effectively induce the synthesis and accumulation of phytoalexins; serve as early informational molecules that are of great significance for plants in: the resistance to diseases and infection, molecular signal regulation, growth and development, morphogenesis, adaptation to the environment and other aspects; and have favorable prevention and treatment effects on wheat powdery mildew, potato late blight, etc. (4) Oligogalacturonic acids: oligosaccharides composed of 2-20 galacturonic acids linked by α-1,4-glycosidic bonds, derived from pectic polysaccharides and widely distributed in primary cell walls and intercellular layers of roots, stems, leaves and fruits of higher plants; play a role in softening and adhesion of cell tissues; and have favorable prevention and treatment effects on pepper virus and apple mosaic virus diseases.

Low-DP β-1,3-glucooligosaccharides prepared from laminarin have been proved to have a favorable disease prevention effect on various plants, and relevant products have been marketed. However, oligosaccharides produced from laminarin have a low DP and are not uniform due to limitations of sources of raw materials and the hydrolysis process, making it difficult to give full play to their functions. Therefore, there is an urgent need to find an ideal alternative raw material to efficiently prepare oligosaccharides with a high DP and a uniform structure, and to explore the plant induction mechanism of new oligosaccharides.

YANG YUNXIA ET AL, "Anti-tumor activity and immunogenicity of a succinoglycan ridin", CARBOHYDRATE POLYMERS, APPLIED SCIENCE PUBLISHERS, LTD BARKING, GB, vol. 255, doi:10.1016/J.CARBPOL.2020.117370, ISSN 0144-8617, (20201112), (20201112), XP086440893 describes antitumor effects of riclin being a succinoglycan. CHOULY ET AL, "NMR studies of succinoglycan repeating-unit octasaccharides from Rhizobium meliloti and Agrobacterium radiobacter", INTERNATIONAL JOURNAL OF BIOLOGICAL MACROMOLECULES, ELSEVIER BV, NL, (19950101), vol. 17, no. 6, doi:10.1016/0141-8130(96)81846-0, ISSN 0141-8130, pages 357 - 363, XP005295703 describes $^1H$ and $^{13}C$-nuclear magnetic resonance assignments for determination of locations of the O-succinyl and O-acetyl substituents. CHENG R. ET AL, "In vitro and in vivo anti-inflammatory activity of a succinoglycan Riclin from Agrobacterium sp. ZCC3656", JOURNAL OF APPLIED MICROBIOLOGY, GB, vol. 127, no. 6, doi:10.1111/jam.14447, ISSN 1364-5072, (20191021), pages 1716 - 1726, URL: https://onlinelibrary.wiley.com/doi/full-xml/10.1111/jam.14447, XP093125170 describes Agrobacterium sp. ZCC3656 being a highly stable EPS-producing strain. FU RENJIE ET AL, "Transcriptomic and metabolomic profiling revealed the role of succinoglycan Ridin octaose in eliciting the defense response of Solanum tuberosum", APPLIED MICROBIOLOGY AND BIOTECHNOLOGY, SPRINGER BERLIN HEIDELBERG, BERLIN/HEIDELBERG, vol. 105, no. 19, doi:10.1007/S00253-021-11588-1, ISSN 0175-7598, (20210918), pages 7439 - 7450, (20210918), XP037582744 describes a homogeneous octaose which was depolymerized from the succinoglycan Riclin and was investigated as a novel elicitor to activate the immune system of potato (Solanum tuberosum L.).

## SUMMARY

The present invention aims to provide an application of a heteropoly oligosaccharide in improving disease resistance of a plant.

The heteropoly oligosaccharide contains seven D-glucose residues and one D-galactose residue, and the structure thereof is represented as follows:

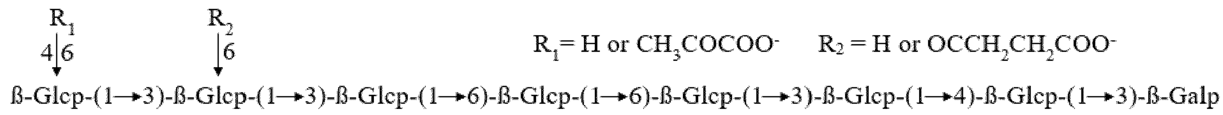

$R_1$ = H or $CH_3COCOO^-$ $R_2$ = H or $OCCH_2CH_2COO^-$

.

$R_1$ is H or a monomolecular pyruvate group, and $R_2$ is H or a monomolecular succinyl group.

In an embodiment not falling under the scope of protection, the heteropoly oligosaccharide of the present invention is prepared through enzymolysis of an exopolysaccharide Riclin under an action of β-glucanase, the exopolysaccharide Riclin being produced from Agrobacterium sp. ZCC3656 (CCTCC No.: M 2018797). Details are given below:

(1) Enzymolysis of polysaccharide Riclin:

An exopolysaccharide Riclin produced from Agrobacterium sp. ZCC3656 (CCTCC No.: M 2018797) is dissolved in an aqueous solution, β-glucanase is added to obtain a mixture. The mixture is placed in a thermostatic water bath at 60°C for reaction and vibrated until the exopolysaccharide Riclin is enzymolyzed completely.

(2) Purification of heteropoly oligosaccharide:

A solution obtained from the enzymolysis in step (1) is centrifuged to remove insoluble impurities and obtain a supernatant. A mixed solvent of chloroform and n-butanol at a volume ratio of 4:1 is added to the supernatant to remove proteins from the supernatant. The solution is vibrated vigorously and left to stand for stratification, the aqueous phase is taken and centrifuged to remove the protein layer, and the supernatant is retained. These steps are repeated several times until the protein layer is removed completely. Finally, 95% ethanol is added to precipitate a heteropoly oligosaccharide.

The Agrobacterium sp. ZCC3656 has been disclosed in CN 111 286 466 A.

In an embodiment of the present invention, the plants include but are not limited to crops and other plants, such as tobacco, wheat, tomato, potato, apple, strawberry, paddy, and soybean.

In an embodiment of the present invention, for the application of the heteropoly oligosaccharide in improving plant disease resistance, the plant disease resistance is the resistance to pathogenic bacteria infection in plants, including but not limited to the disease resistance to mosaic virus infection, disease resistance to *Fusarium graminearum* infection, disease resistance to *Cladosporium fulvum* infection, disease resistance to *Phytophthora infestans* infection, disease resistance to *Marssonina mali* infection and disease resistance to *Pseudomonas solanacearum* infection.

The present disclosure also provides a plant disease resistance inducer with an active ingredient containing the heteropoly oligosaccharide not falling under the scope of protection.

In the plant disease resistance inducer of the present disclosure, a concentration of the heteropoly oligosaccharide is 5-5,000 mg/L, and preferably 50-200 mg/L.

The heteropoly oligosaccharide of the present disclosure applied to crops and various other plants as a plant

disease resistance inducer can significantly improve plant disease resistance, specifically as follows. 1) When acting on tobacco leaves, it can obviously increase the concentration of hydrogen peroxide in tobacco leaves as the dose increases; 2) When acting on lower epidermal cells of wheat leaves, it can obviously induce the release of hydrogen peroxide from cells of wheat leaves as the fluorescence intensity increases; 3) When acting on tomato leaves, it can obviously improve activities of glucanase, chitinase and phenylalanine ammonia lyase continuously as the dose increases and the induction time prolongs; 4) When acting on potato leaves infected with Phytophthora infestans patches, it can obviously reduce the damage of pathogenic bacteria to plants, and alleviate and even eliminate pathogenic bacteria infection.

Compared with the prior art, the present invention has the following advantages:

(1) The heteropoly oligosaccharide of the present disclosure can induce plants to produce the resistance to pathogenic bacteria infection with a significant induction effect, thereby obviously reducing occurrences of pathogenic bacteria infection in plants.

(2) A method of externally applying the heteropoly oligosaccharide is adopted in the present invention; the application method is simple, no biological toxicity is produced and no environmental pollution is caused.

(3) The heteropoly oligosaccharide of the present disclosure is prepared through strain fermentation by a simple method at a low cost, can be put into mass production and has a uniform structure, thereby having extensive application prospects as a new biological pesticide.

## BRIEF DESCRIPTION OF THE DRAWINGS

FIG. 1 is a diagram showing changes of $H_2O_2$ concentration in tobacco leaves induced by a heteropoly oligosaccharide.

FIG. 2 shows $H_2O_2$ concentrations in cells of wheat leaves induced by the heteropoly oligosaccharide as observed through $H_2$DCF-DA fluorescence detection, where CK is a control group sprayed with water, 50 ppm means that the concentration of the heteropoly oligosaccharide is 50 mg/L, and 500 ppm means that the concentration of the heteropoly oligosaccharide is 500 mg/L.

FIG. 3 shows activities of resistance-related enzymes in tomato leaves induced by the heteropoly oligosaccharide, where a shows activity of glucanase; b shows activity of chitinase, and c shows activity of phenylalanine ammonia lyase.

FIG. 4 shows effects of the heteropoly oligosaccharide on potato leaves against *Phytophthora infestans* infection, where CK is a control group sprayed with water, 50 ppm means that the concentration of the heteropoly oligosaccharide is 50 mg/L, and 500 ppm means that the concentration of the heteropoly oligosaccharide is 500 mg/L.

## DETAILED DESCRIPTION

The present invention will be further described below in detail in conjunction with the specific embodiments and accompanying drawings.

Agrobacterium sp. ZCC3656 used in the following embodiments has been disclosed in CN 111 286 466 A.

Example 1: Screening and purification of heteropoly oligosaccharide.

1. Preparation and separation of polysaccharide

Agrobacterium sp. ZCC3656 was inoculated into an Htm (containing 1 g of sodium dihydrogen phosphate, 0.07 g of anhydrous calcium chloride, 0.2 g of magnesium chloride, 0.0125 g of ferrous sulfate, 3 g of potassium nitrate, 0.003 g of manganese sulfate, 0.0075 g of zinc chloride, 1,000 mL of water and 20 g of sucrose, with a pH value of 7.0-7.2) liquid medium, and cultured in a shaker at 28°C and 250 rpm for 48 h; a 2-fold volume of industrial ethanol (95% ethanol) was added to the fermentation broth, and white filamentous polysaccharide precipitate was observed; the precipitate was collected through centrifugation at 6,000 × g and dried in a vacuum drying oven at 60°C for 8 h, and the solid polysaccharide was pulverized with a grinder to obtain a crude polysaccharide.

2. Preparation of heteropoly oligosaccharide

The polysaccharide was weighed and dissolved in an aqueous solution, β-glucanase was added, the mixture was uniformly mixed and allowed for reaction in a thermostatic water bath at 60°C, and vibrated 1 h later until the polysaccharide solution became not viscous any more.

3. Purification of heteropoly oligosaccharide

The enzymolyzed solution was centrifuged at 9,000 rpm for 30 min to remove insoluble impurities. Proteins were removed from the centrifuged supernatant by a Sevage method, i.e., by adding a 1/4 volume of a chloroform-n-butanol (4:1) mixture, the solution was vibrated vigorously for 30 min and left to stand for stratification, the aqueous phase was centrifuged at 7,000 rpm for 10 min to remove the protein layer, and the supernatant was transferred to a clean container. These operations were repeated several times until there was no protein layer. Finally, a 3-fold volume of 95% ethanol was added to precipitate a heteropoly oligosaccharide, and the heteropoly oligosaccharide precipitate was dried in the vacuum drying oven at 50°C.

Example 2: Application of heteropoly oligosaccharide in improving tobacco mosaic disease resistance

1. Establishment of tobacco test model:

The test tobacco variety was *Nicotiana X sanderae.* The laboratory culture was conducted at a constant temperature of 30°C for 12 h of lightness and 12 h of darkness, and the soil was kept moist. The test was divided into six groups: a control group sprayed with water, and groups sprayed with the heteropoly oligosaccharide at different concentrations (5 mg/L, 25 mg/L, 50 mg/L, 100 mg/L and 250 mg/L, respectively). The leaves were collected within 15 min after spraying to determine relevant signaling molecules.

2. Determination of $H_2O_2$ concentration in tobacco leaves:

100-200 mg of fresh plant leaves were weighed and quickly ground into superfine powder in liquid nitrogen; 1 mL of acetone was added, the mixture was extracted under vortex vibration and centrifuged at 10,000 rpm for 10 min, and the supernatant was transferred to a clean centrifuge tube; 0.8 mL of extract was taken and put in a 2 mL centrifuge tube, 0.1 mL of titanium tetrachloride reagent (5 mL of titanium tetrachloride reagent contains 2.175 mL of concentrated hydrochloric acid, 1 mL of titanium tetrachloride and 1.825 mL of deionized water) was added, then 0.2 mL of concentrated ammonia water was added, the solution was centrifuged at 5,000 rpm for 10 min, and the precipitate was collected; the precipitate was washed with acetone 3-5 times until the precipitate turned white; 1 mL of 2M $H_2SO_4$ was added to the washed precipitate, and the precipitate was dissolved under vibration; 200 μL of sample was transferred to a 96-well colorimetric plate, and the absorbance value at 415 nm was read. The results are shown in FIG. 2.

3. Establishment of tobacco mosaic disease model:

The test tobacco variety was *Nicotiana X sanderae.* The outdoor culture was conducted, and the soil was kept moist. The test was divided into five groups: a control group sprayed with water, and groups sprayed with the heteropoly oligosaccharide at different concentrations (50 mg/L, 100 mg/L, 200 mg/L and 500 mg/L, respectively). A tobacco mosaic virus was evenly sprayed on surfaces of tobacco leaves two days after the heteropoly oligosaccharide was sprayed, and the leaves were observed for infection. After the tobacco mosaic virus was sprayed, infection conditions of tobacco leaves were recorded every other week for three consecutive weeks. The results are shown in Table 1.

Table 1 Application of heteropoly oligosaccharide in improving tobacco mosaic disease resistance

| Statistics of preventive test before onset of tobacco mosaic disease | | | | | | |
|---|---|---|---|---|---|---|
| Concentration of heteropoly oligosaccharide (mg/L) | One week after spraying | | Two weeks after spraying | | Three weeks after spraying | |
| | Control effect (%) | Incidence (%) | Control effect (%) | Incidence (%) | Control effect (%) | Incidence (%) |
| 0 | | 10.47 | | 38.31 | | 68.32 |
| 50 | 50.56 | 2.51 | 62.58 | 11.83 | 81.36 | 12.73 |
| 100 | 62.37 | 1.86 | 75.44 | 6.39 | 84.59 | 10.53 |

(continued)

| Statistics of preventive test before onset of tobacco mosaic disease | | | | | | |
|---|---|---|---|---|---|---|
| Concentration of heteropoly oligosaccharide (mg/L) | One week after spraying | | Two weeks after spraying | | Three weeks after spraying | |
| | Control effect (%) | Incidence (%) | Control effect (%) | Incidence (%) | Control effect (%) | Incidence (%) |
| 200 | 60.31 | 1.31 | 79.39 | 4.72 | 90.21 | 6.69 |
| 500 | 67.62 | 0.89 | 82.54 | 3.39 | 94.86 | 3.51 |

4. Data analysis

To compare different groups, statistical analysis was conducted by an ANOVA statistical method. $P<0.05$ was considered to indicate a significant difference.

It can be seen from FIG. 1 and Table 1 that the concentration of hydrogen peroxide in tobacco leaves obviously increased after the heteropoly oligosaccharide was sprayed. The concentration of hydrogen peroxide increased linearly and dependently as the concentration of the heteropoly oligosaccharide increased. According to the mosaic virus infection conditions of tobacco, the heteropoly oligosaccharide can obviously improve the resistance of tobacco to mosaic virus, enhance prevention and treatment effects on tobacco against the virus, and reduce the infection rate of tobacco leaves. Thus, the heteropoly oligosaccharide can be a potential biological pesticide for improving plant disease resistance.

Example 3: Application of heteropoly oligosaccharide in improving wheat scab resistance

1. Establishment of wheat test model:

The test wheat variety was Miannong No. 6. The laboratory culture was conducted at a constant temperature of 25°C for 12 h of lightness and 12 h of darkness, and the soil was kept moist.

2. Fluorescence detection of hydrogen peroxide in wheat leaves

The test was divided into three groups: a blank control group (i.e., using water as a control), a low-concentration heteropoly oligosaccharide group (with a final oligosaccharide concentration of 50 mg/L), and a high-concentration heteropoly oligosaccharide group (with a final oligosaccharide concentration of 500 mg/L). The fluorescence detection of $H_2O_2$ was conducted by the following method: $H_2$DCF-DA was prepared into a 1 mmol/L stock solution by DMSO, lower epidermis was carefully torn from plant leaves and incubated in a citric acid buffer with a pH value of 5.6 for 4 h, then the citric acid buffer was replaced, $H_2$DCF-DA was added until the final concentration reached 10 μmol/L, and incubation was conducted under horizontal shaking for 30 min; upon completion of incubation, excess fluorescent dye was removed through 3 times of rinsing with the citric acid buffer; the lower epidermis of leaves was placed on a glass slide, and the fluorescence intensity was observed using a BP460-490 laser color filter within a light wavelength range of 460-490 nm. The results are shown in FIG. 2.

3. Establishment of wheat scab model:

The test wheat variety was Miannong No. 6. The outdoor culture was conducted, and the soil was kept moist. The test was divided into five groups: a control group sprayed with water, and groups sprayed with the heteropoly oligosaccharide at different concentrations (50 mg/L, 100 mg/L, 200 mg/L and 500 mg/L, respectively). A *Fusarium graminearum* spore suspension was evenly sprayed on surfaces of wheat leaves two days after the heteropoly oligosaccharide was sprayed, and the leaves were observed for infection. Infection conditions of wheat leaves were recorded two weeks after the *Fusarium graminearum* spore suspension was sprayed. The results are shown in Table 2.

$$\text{Incidence} = \text{number of diseased plants}/\text{number of investigated plants} \times 100\%;$$

Disease index = Σ (number of diseased leaves at all levels × value of corresponding level)/(total number of investigated leaves × representative value of highest level) × 100;

Control effect = (disease index of control group - disease index of treatment group) / disease index of control group × 100%.

Table 2 Application of heteropoly oligosaccharide in improving wheat scab resistance

| Statistics of preventive test before onset of wheat scab | | | |
|---|---|---|---|
| Concentration of heteropoly oligosaccharide (mg/L) | Incidence (%) | Disease index | Control effect (%) |
| 0 | 32.1 | 40.6 | |
| 50 | 6.4 | 10.5 | 74.1 |
| 100 | 6.2 | 8.7 | 78.6 |
| 200 | 4.6 | 6.9 | 83.0 |
| 500 | 3.7 | 3.7 | 90.9 |

4. Data analysis

To compare different groups, statistical analysis was conducted by an ANOVA statistical method. $P<0.05$ was considered to indicate a significant difference.

It can be seen from FIG. 2 and Table 2 that the fluorescence intensity of hydrogen peroxide produced by stomatal cells was low when lower epidermal cells of wheat leaves were not induced by the heteropoly oligosaccharide, and the heteropoly oligosaccharide with a final concentration of 500 mg/L can obviously induce stomatal cells to produce a large amount of hydrogen peroxide. According to the scab infection conditions of wheat, spraying the heteropoly oligosaccharide can improve the resistance of wheat to *Fusarium graminearum* spores. After the heteropoly oligosaccharide was sprayed, the control effect significantly increased and the incidence obviously decreased. Thus, the heteropoly oligosaccharide can be a potential biological pesticide for improving plant disease resistance.

Example 4: Application of heteropoly oligosaccharide in improving tomato leaf mold resistance

1. Establishment of tomato test model

The test tomato variety was Huizhen No. 1. The laboratory culture was conducted at a constant temperature of 25°C for 12 h of lightness and 12 h of darkness, and the soil was kept moist.

2. Activity determination of enzymes related to tomato leaf resistance

The test was divided into three groups: a blank control group (i.e., using water as a control), a low-concentration heteropoly oligosaccharide group (with a final oligosaccharide concentration of 50 mg/L), and a high-concentration heteropoly oligosaccharide group (with a final oligosaccharide concentration of 150 mg/L). Samples were taken 4 h, 24 h and 72 h after the heteropoly oligosaccharide was sprayed to determine activities of relevant proteases.

(1) Extraction of crude enzyme: 25 mg of leaves were quickly ground into superfine powder in liquid nitrogen, and 500 μL of protein extract (containing a 20 mM PBS buffer, 5 mM EDTA2 Na, 2.5 mM DTT, 2.5 mM $Na_2S_2O_3 \cdot 5H_2O$ and 0.5% pvp) was added.

(2) Activity determination of glucanase: A reaction solution contained 200 μL of 1% laminarin, 100 μL of crude enzyme extract, and 200 μL of 50 mM sodium acetate buffer with a pH value of 5.0 which were mixed uniformly, the mixture was allowed for reaction at 37°C for 2 h and then put in a boiling water bath for 10 min, and the reaction was terminated; 100 μL of enzymolysis solution was taken, 100 μL of DNS was added, the resulting solution was treated in the boiling water bath for 5 min and then cooled in an ice water bath, and the absorbance value at 540 nm was read.

(3) Activity determination of chitinase: A reaction solution contained 200 μL of chitin substrate, 100 μL of crude enzyme extract, and 200 μL of 50 mM sodium acetate buffer with a pH value of 5.0 which were mixed uniformly, the mixture was allowed for reaction at 37°C for 2 h and then put in a boiling water bath for 10 min, and the reaction was terminated; 100 μL of enzymolysis solution was taken, 100 μL of DNS was added, the resulting solution was treated in the boiling water bath for 5 min and then cooled in an ice water bath, and the absorbance value at 540 nm was read.

(4) Activity determination of phenylalanine ammonia lyase: A reaction solution contained 100 μL of crude enzyme extract, 500 μL of 20 mM L-phenylalanine solution, and 400 μL of 50 mM Tris-HCl buffer with a pH value of 8.5, and reacted at 37°C for 2 h; 0.2 mL of 6M HCl was added to terminate the reaction, 2 mL of ether was added to extract cinnamic acid, and the solution was centrifuged at 3,000 rpm for 1 min; the extractant was transferred to a clean 5 mL centrifuge tube, the extraction was repeated once, and extracts were put together and dried with nitrogen; 0.5 mL of methanol was added for redissolution, and the absorbance value at 290 nm was read. The results are shown in FIG. 3.

3. Establishment of tomato leaf mold model

The test tomato variety was Huizhen No. 1. The outdoor culture was conducted, and the soil was kept moist. The test was divided into five groups: a control group sprayed with water, and groups sprayed with the heteropoly oligosaccharide at different concentrations (50 mg/L, 100 mg/L, 200 mg/L and 500 mg/L, respectively). A *Cladosporium fulvum* spore suspension was evenly sprayed on surfaces of tomato leaves two days after the heteropoly oligosaccharide was sprayed, and the leaves were observed for infection. Infection conditions of tomato leaves were recorded two weeks after the *Cladosporium fulvum* spore suspension was sprayed. The results are shown in Table 3.

$$\text{Incidence} = \text{number of diseased plants}/\text{number of investigated plants} \times 100\%;$$

Disease index = Σ (number of diseased leaves at all levels × value of corresponding level)/(total number of investigated leaves × representative value of highest level) × 100;

Control effect = (disease index of control group - disease index of treatment group)/disease index of control group × 100%.

Table 3 Application of heteropoly oligosaccharide in improving tomato leaf mold resistance

| Statistics of preventive test before onset of tomato leaf mold | | | |
|---|---|---|---|
| Concentration of heteropoly oligosaccharide (mg/L) | Incidence (%) | Disease index | Control effect (%) |
| 0 | 42.6 | 58.7 | |
| 50 | 8.5 | 12.6 | 78.5 |
| 100 | 7.2 | 11.9 | 79.7 |
| 200 | 5.7 | 8.8 | 85.0 |
| 500 | 2.3 | 5.7 | 90.3 |

4. Data analysis

To compare different groups, statistical analysis was conducted by an ANOVA statistical method. $P<0.05$ was considered to indicate a significant difference.

It can be seen from FIG. 3 and Table 3 that after the heteropoly oligosaccharide was sprayed onto tomato leaves, the activity of chitinase significantly increased at 72 h, while activities of glucanase and phenylalanine ammonia lyase significantly increased at 24 h. Spraying the heteropoly oligosaccharide to tomato can improve the resistance of tomato to *Cladosporium fulvum* spores. After the heteropoly oligosaccharide was sprayed, the control effect significantly increased and the incidence obviously decreased. Thus, the heteropoly oligosaccharide can be a potential biological pesticide for improving plant disease resistance.

Example 5: Application of heteropoly oligosaccharide in improving potato *Phytophthora* disease resistance

1. Establishment of potato test model

The test potato variety was Fovorita. The laboratory culture was conducted at a constant temperature of 25°C for 12 h of lightness and 12 h of darkness, and the soil was kept moist.

2. Test of potato Phytophthora *infestans* infection

The test was divided into three groups: a blank control group (i.e., using water as a control), a low-concentration heteropoly oligosaccharide group (with a final oligosaccharide concentration of 50 mg/L), and a high-concentration heteropoly oligosaccharide group (with a final oligosaccharide concentration of 500 mg/L). Before the test, potato leaves were placed on a flat plate, and petioles were kept wet to prevent leaves from withering. The leaves were sprayed with different concentrations of the heteropoly oligosaccharide, kept wet and cultured at 20°C for 36 h. *Phytophthora infestans* patches were attached onto potato leaves. The leaves were kept away from light for 24 h, and cultured for 12 h of lightness and 12 h of darkness. Two days later, infection conditions of leaves with patches were observed. The results are shown in FIG. 4.

3. Field test of potato Phytophthora *infestans* infection

The test was divided into four groups: a blank control group (i.e., using water as a control), a low-concentration heteropoly oligosaccharide group (with a final oligosaccharide concentration of 50 mg/L), a medium-concentration heteropoly oligosaccharide group (with a final oligosaccharide concentration of 250 mg/L), and a high-concentration heteropoly oligosaccharide group (with a final oligosaccharide concentration of 500 mg/L). After a *Phytophthora infestans* spore suspension was sprayed, infection conditions of potato leaves were recorded every other week for three consecutive weeks.

4. Data analysis

It can be seen from FIG. 4 and Table 4 that after the heteropoly oligosaccharide was sprayed onto potato leaves, under the condition of infection with pathogenic bacteria patches, severe leaf infection was observed from the control group not treated with the heteropoly oligosaccharide, while leaves sprayed with the heteropoly oligosaccharide were slightly infected or even not infected. Spraying the heteropoly oligosaccharide to potato can improve the resistance of potato to *Phytophthora infestans* infection. After the heteropoly oligosaccharide was sprayed, the control effect significantly increased and the incidence significantly decreased. Thus, the oligosaccharide can favorably induce potato leaves to produce resistance and improve disease resistance, and can be a potential biological pesticide.

Table 4 Application of heteropoly oligosaccharide in improving potato late blight resistance

| Statistics of preventive test before onset of potato late blight | | | | | | |
|---|---|---|---|---|---|---|
| Concentration of heteropoly oligosaccharide (mg/L) | One week after spraying | | Two weeks after spraying | | Three weeks after spraying | |
| | Control effect (%) | Incidence (%) | Control effect (%) | Incidence (%) | Control effect (%) | Incidence (%) |
| 0 | | 26.53 | | 42.31 | | 72.32 |
| 50 | 60.21 | 4.54 | 79.42 | 8.71 | 87.54 | 12.51 |
| 250 | 63.56 | 3.32 | 82.67 | 7.33 | 90.21 | 10.69 |
| 500 | 70.39 | 0.82 | 87.39 | 5.34 | 95.36 | 7.17 |

Example 6: Application of heteropoly oligosaccharide in improving apple brown spot resistance

1. Establishment of apple test model

The test apple variety was Red Fuji. The outdoor culture was conducted, and the soil was kept moist. The test was performed during a lush growth of leaves and before apples were borne.

2. Test of *Marssonina mali* infection

The test was divided into three groups: a blank control group (i.e., using water as a control), a low-concentration heteropoly oligosaccharide group (with a final oligosaccharide concentration of 50 mg/L), and a high-concentration heteropoly oligosaccharide group (with a final oligosaccharide concentration of 500 mg/L). A *Marssonina mali* spore suspension was evenly sprayed on surfaces of apple tree leaves two days after the heteropoly oligosaccharide was sprayed, and the leaves were observed for infection. After the *Marssonina mali* spore suspension was sprayed, infection

conditions of apple tree leaves were recorded every other week for three consecutive weeks. The results are shown in Table 5.

Table 5 Application of heteropoly oligosaccharide in improving apple brown spot resistance

| Statistics of preventive test before onset of apple brown spot | | | | | | |
|---|---|---|---|---|---|---|
| Concentration of heteropoly oligosaccharide (mg/L) | One week after spraying | | Two weeks after spraying | | Three weeks after spraying | |
| | Control effect (%) | Incidence (%) | Control effect (%) | Incidence (%) | Control effect (%) | Incidence (%) |
| 0 | | 31.34 | | 50.13 | | 74.02 |
| 50 | 68.54 | 7.12 | 74.87 | 12.60 | 84.79 | 14.65 |
| 500 | 76.77 | 4.21 | 85.32 | 7.36 | 93.21 | 8.75 |

3. Data analysis

It can be seen from Table 5 that after the heteropoly oligosaccharide was sprayed onto apple tree leaves, under the condition of infection with the pathogenic bacteria spore suspension, severe leaf infection was observed from the control group not treated with the heteropoly oligosaccharide, while leaves sprayed with the heteropoly oligosaccharide were slightly or even not infected. Spraying the heteropoly oligosaccharide to apple trees can improve the resistance of apple trees to *Marssonina mali.* After the heteropoly oligosaccharide was sprayed, the incidence obviously decreased. Thus, the heteropoly oligosaccharide can be a potential biological pesticide for improving plant disease resistance.

Example 7: Application of heteropoly oligosaccharide in improving strawberry bacterial wilt resistance

1. Establishment of strawberry test model

The test strawberry variety was Ningxin. The outdoor culture was conducted, and the soil was kept moist. The test was performed during a lush growth of leaves and before strawberries were borne.

2. Test of strawberry *Pseudomonas solanacearum* infection

The test was divided into five groups: a control group sprayed with water, and groups sprayed with the heteropoly oligosaccharide at different concentrations (50 mg/L, 100 mg/L, 200 mg/L and 500 mg/L, respectively). A strawberry *Pseudomonas solanacearum* suspension was evenly sprayed on surfaces of strawberry leaves two days after the heteropoly oligosaccharide was sprayed, and the leaves were observed for infection. Infection conditions of strawberry leaves were recorded two weeks after the *Pseudomonas solanacearum* suspension was sprayed. The results are shown in Table 6.

$$\text{Incidence} = \text{number of diseased plants}/\text{number of investigated plants} \times 100\%;$$

Disease index = Σ (number of diseased leaves at all levels × value of corresponding level)/(total number of investigated leaves × representative value of highest level) × 100;

Control effect = (disease index of control group - disease index of treatment group)/disease index of control group × 100%.

Table 6 Application of heteropoly oligosaccharide in improving strawberry bacterial wilt resistance

| Statistics of preventive test before onset of strawberry bacterial wilt | | | |
|---|---|---|---|
| Concentration of heteropoly oligosaccharide (mg/L) | Incidence (%) | Disease index | Control effect (%) |
| 0 | 37.8 | 58.7 | |
| 50 | 6.8 | 14.4 | 75.5 |

(continued)

| Statistics of preventive test before onset of strawberry bacterial wilt | | | |
|---|---|---|---|
| Concentration of heteropoly oligosaccharide (mg/L) | Incidence (%) | Disease index | Control effect (%) |
| 100 | 5.2 | 10.7 | 81.8 |
| 200 | 5.1 | 10.2 | 82.6 |
| 500 | 2.3 | 6.6 | 88.8 |

3. Data analysis

It can be seen from Table 6 that after the heteropoly oligosaccharide was sprayed onto strawberry leaves, under the condition of infection with the *Pseudomonas solanacearum* suspension, severe leaf infection was observed from the control group not treated with the heteropoly oligosaccharide, while leaves sprayed with the heteropoly oligosaccharide were slightly or even not infected. Spraying the heteropoly oligosaccharide to strawberries can improve the resistance of strawberries to *Pseudomonas solanacearum.* After the heteropoly oligosaccharide was sprayed, the incidence obviously decreased. Thus, the heteropoly oligosaccharide can be a potential biological pesticide for improving plant disease resistance.

## Claims

1. An application of a heteropoly oligosaccharide in improving disease resistance of a plant, wherein the heteropoly oligosaccharide comprises seven D-glucose residues and one D-galactose residue, **characterized in that** a structure of the heteropoly oligosaccharide molecule is represented as follows:

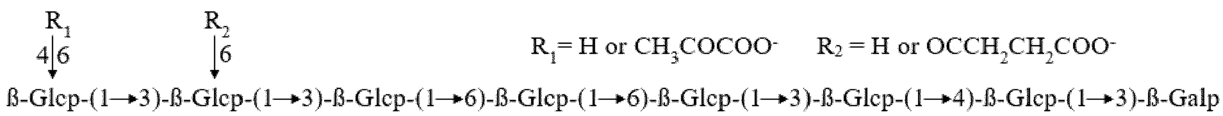

wherein $R_1$ is H or a monomolecular pyruvate group, and $R_2$ is H or a monomolecular succinyl group.

2. The application according to claim 1, **characterized in that**, the plant is tobacco, wheat, tomato, potato, apple, strawberry, paddy or soybean.

3. The application according to claim 1, **characterized in that**, the disease resistance of the plant is resistance to pathogenic bacteria infection in the plant.

4. The application according to claim 1, **characterized in that**, the disease resistance of the plant is disease resistance to mosaic virus infection, disease resistance to *Fusarium graminearum* infection, disease resistance to *Cladosporium fulvum* infection, disease resistance to *Phytophthora infestans* infection, disease resistance to *Marssonina mali* infection, or disease resistance to *Pseudomonas solanacearum* infection.

## Patentansprüche

1. Anwendung eines Heteropolyoligosaccharids zum Verbessern einer Krankheitsresistenz einer Pflanze, wobei das Heteropolyoligosaccharid sieben D-Glucose-Reste und einen D-Galactose-Rest umfasst, **dadurch gekennzeichnet, dass** eine Struktur des Heteropolyoligosaccharidmoleküls wie folgt dargestellt wird:

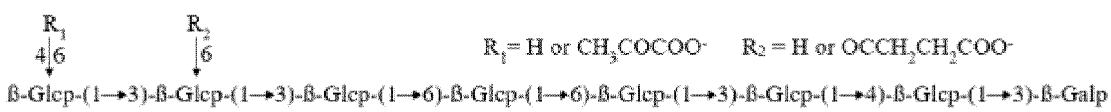

wobei $R_1$ H oder eine monomolekulare Pyruvat-Gruppe ist und $R_2$ H oder eine monomolekulare Succinyl-Gruppe ist.

2. Anwendung nach Anspruch 1, **dadurch gekennzeichnet, dass** die Pflanze Tabak, Weizen, Tomate, Kartoffel, Apfel, Erdbeere, Reis oder Sojabohne ist.

3. Anwendung nach Anspruch 1, **dadurch gekennzeichnet, dass** die Krankheitsresistenz der Pflanze eine Resistenz gegen pathogene Bakterieninfektion der Pflanze ist.

4. Anwendung nach Anspruch 1, **dadurch gekennzeichnet, dass** die Krankheitsresistenz der Pflanze eine Krankheitsresistenz gegen Mosaikvirusinfektion, Krankheitsresistenz gegen eine Infektion mit *Fusarium graminearum,* Krankheitsresistenz gegen eine Infektion mit *Cladosporium fulvum,* Krankheitsresistenz gegen eine Infektion mit *Phytophthora infestans,* Krankheitsresistenz gegen eine Infektion mit *Marssonina mali* oder Krankheitsresistenz gegen eine Infektion mit *Pseudomonas solanacearum* ist.

## Revendications

1. Application d'un hétéropoly-oligosaccharide dans l'amélioration de la résistance d'une plante aux maladies, dans laquelle l'hétéropoly-oligosaccharide comprend sept résidus de D-glucose et un résidu de D-galactose, **caractérisée en ce qu'**une structure de la molécule d'hétéropoly-oligosaccharide est représentée comme suit :

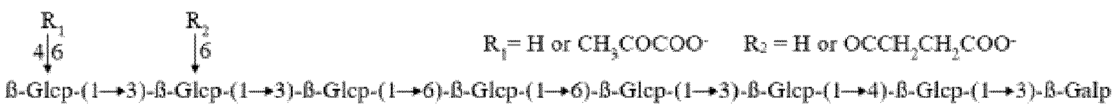

où $R_1$ est H ou un groupe pyruvate monomoléculaire, et $R_2$ est H ou un groupe succinyle monomoléculaire.

2. Application selon la revendication 1, **caractérisée en ce que** la plante est du tabac, du blé, une tomate, une pomme de terre, une pomme, une fraise, du paddy ou du soja.

3. Application selon la revendication 1, **caractérisée en ce que** la résistance de la plante aux maladies est une résistance à une infection bactérienne pathogène dans la plante.

4. Application selon la revendication 1, **caractérisée en ce que** la résistance de la plante aux maladies est une résistance aux maladies d'infection par le virus de la mosaïque, une résistance aux maladies d'infection *Fusarium graminearum,* une résistance aux maladies d'infection *Cladosporium fulvum,* une résistance aux maladies d'infection *Phytophthora infestans,* une résistance aux maladies d'infection *Marssonina mali,* ou une résistance aux maladies d'infection *Pseudomonas solanacearum.*

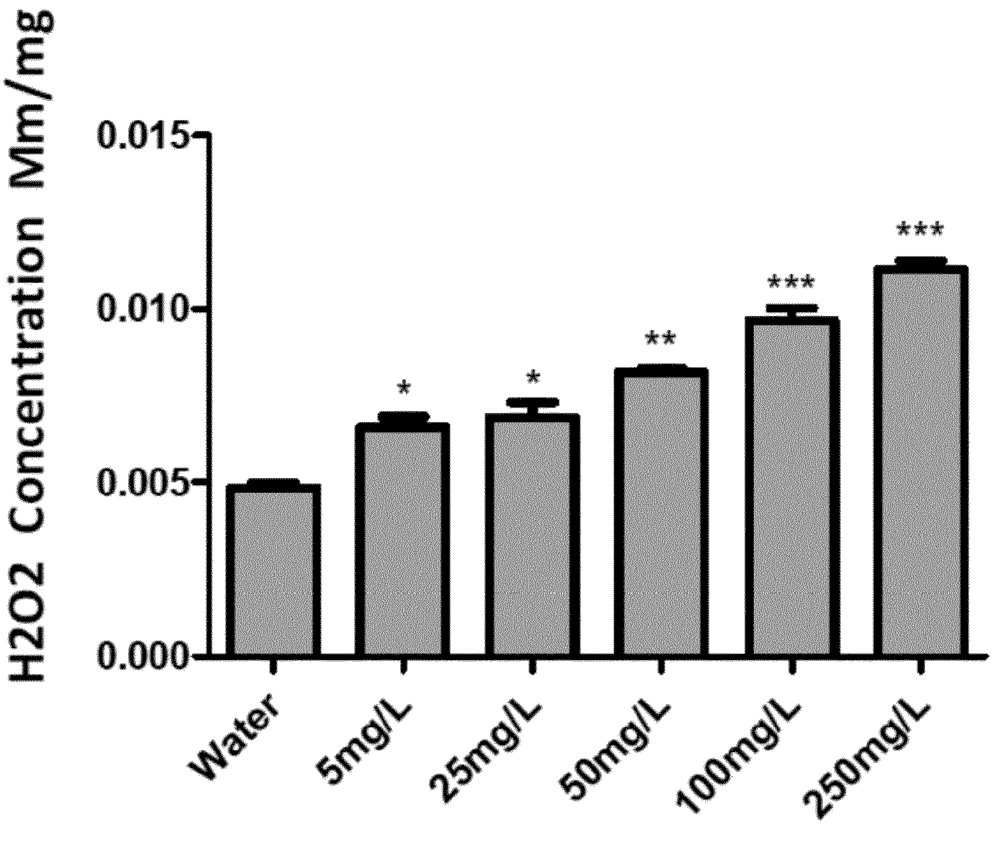
H2O2 Concentration Mm/mg
0.015
0.010
0.005
0.000
*
*
**
***
***
Water
5mg/L
25mg/L
50mg/L
100mg/L
250mg/L

FIG. 1

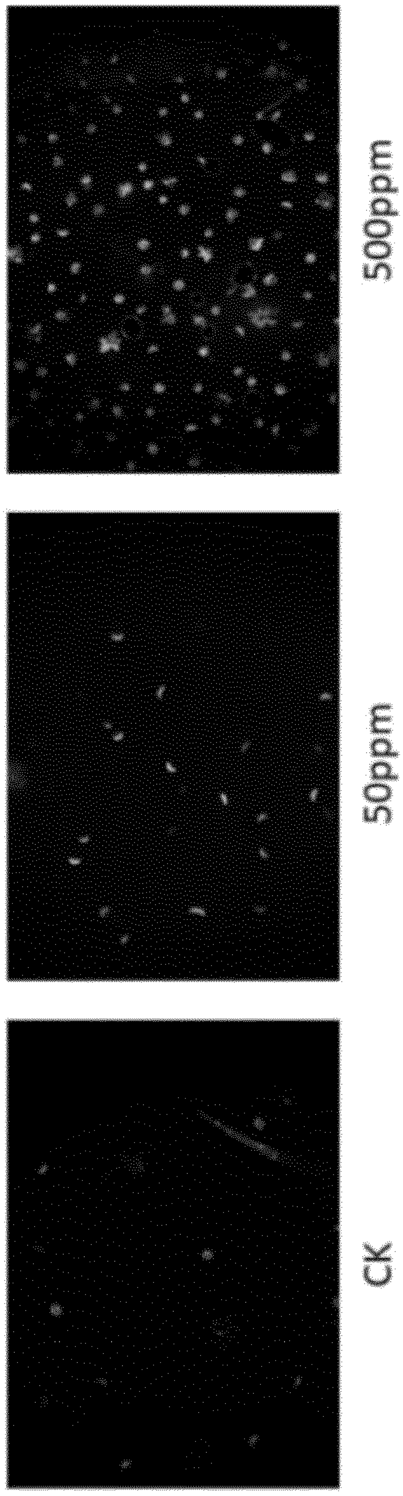


FIG. 2

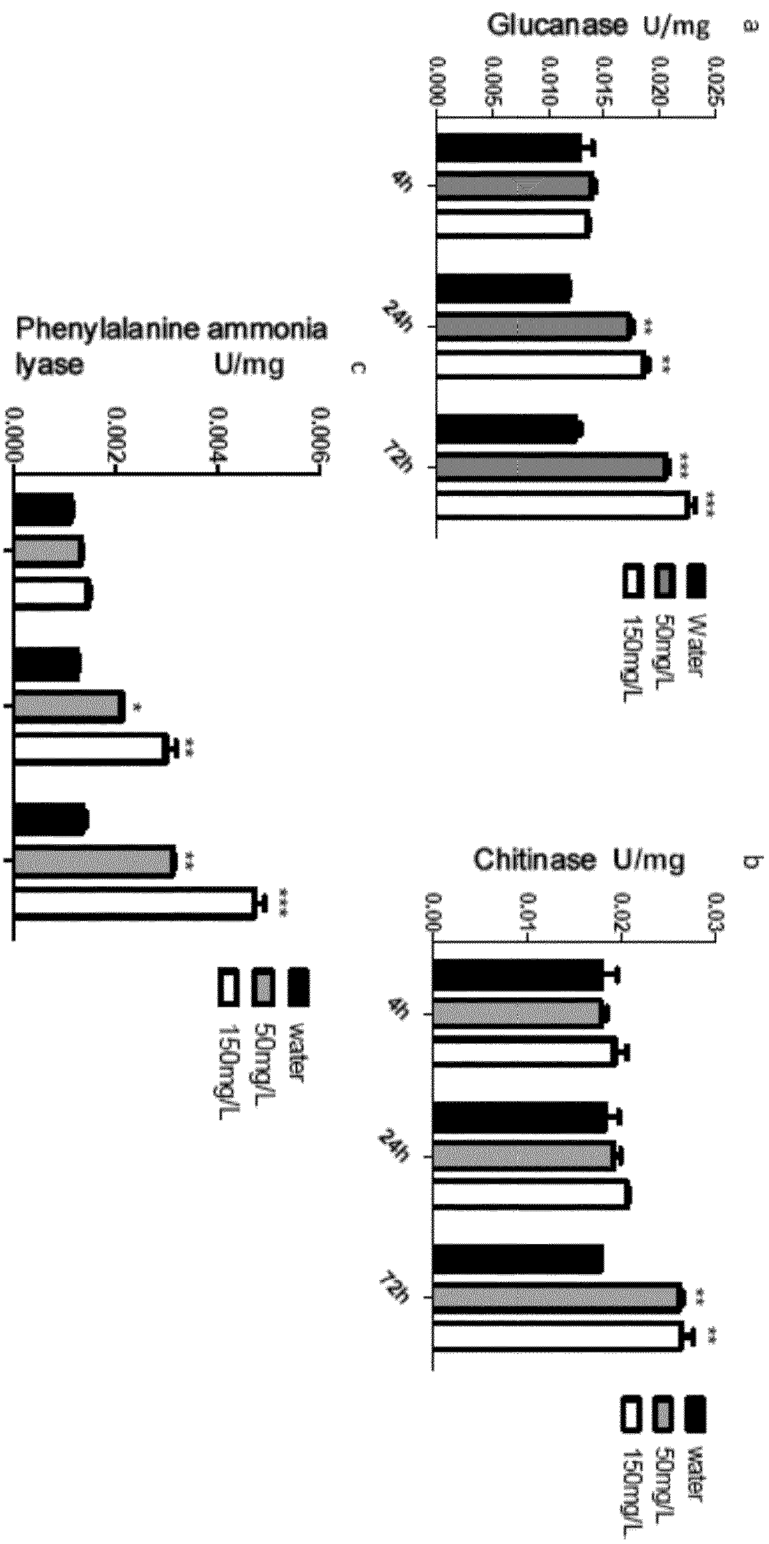
a
Glucanase U/m
0.000
0.005
0.010
0.015
0.020
0.025
4h
24h
72h
Water
50mg/L
150mg/L
b
Chitinase U/mg
0.00
0.01
0.02
0.03
4h
24h
72h
water
50mg/L
150mg/L
c
Phenylalanine ammonia lyase U/mg
0.000
0.002
0.004
0.006
4h
24h
72h
water
50mg/L
150mg/L

FIG. 3

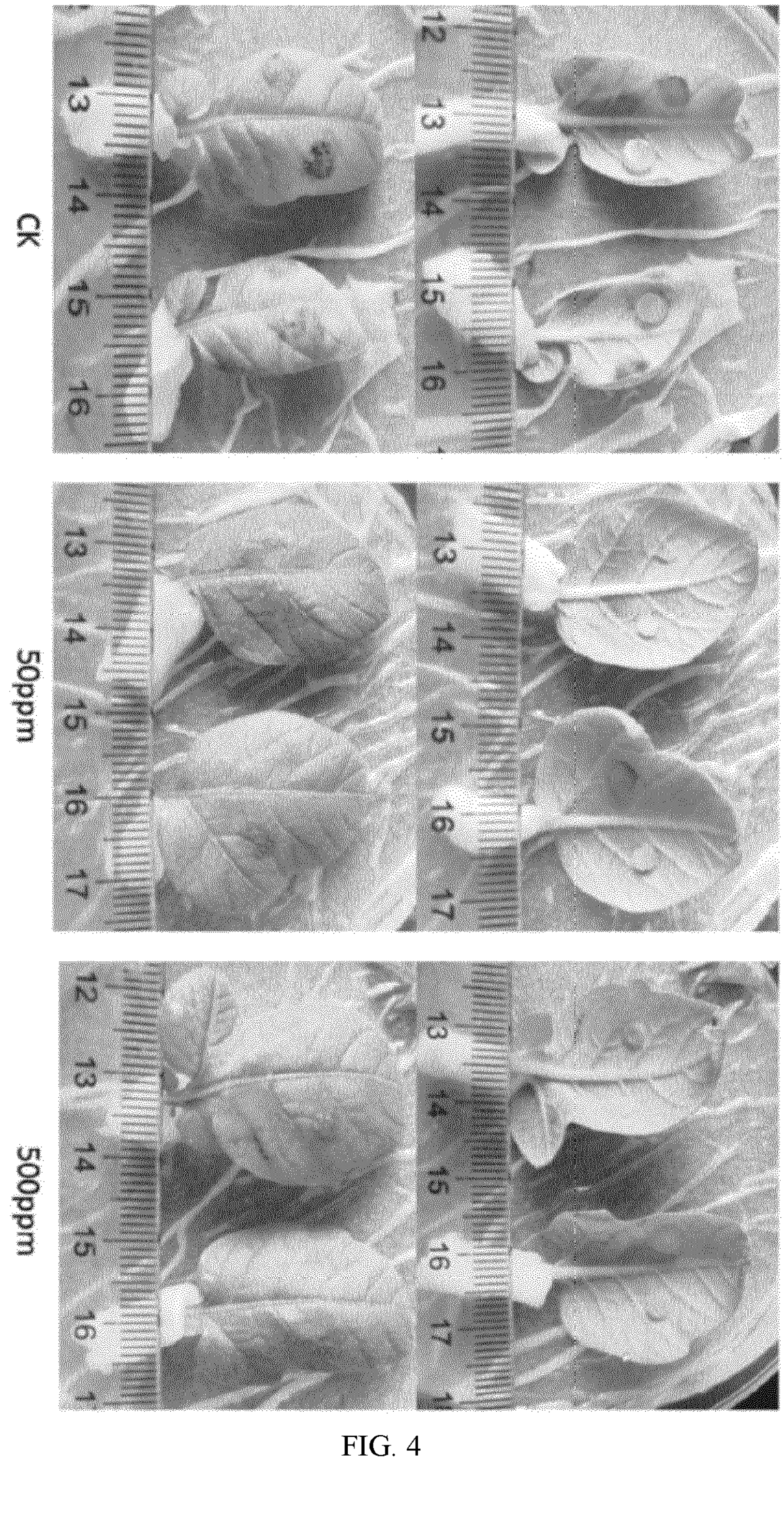


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111286466 A


### Non-patent literature cited in the description


- Anti-tumor activity and immunogenicity of a succinoglycan ridin. **YANG YUNXIA et al.** CARBOHYDRATE POLYMERS. APPLIED SCIENCE PUBLISHERS, LTD, vol. 255
- NMR studies of succinoglycan repeating-unit octasaccharides from Rhizobium meliloti and Agrobacterium radiobacter. **CHOULY et al.** INTERNATIONAL JOURNAL OF BIOLOGICAL MACROMOLECULES. ELSEVIER BV, vol. 17, 357-363
- **CHENG R. et al.** In vitro and in vivo anti-inflammatory activity of a succinoglycan Riclin from Agrobacterium sp. ZCC3656. *JOURNAL OF APPLIED MICROBIOLOGY*, vol. 127, 1716-1726, https://onlinelibrary.wiley.com/doi/full-xml/10.1111/jam.14447
- Transcriptomic and metabolomic profiling revealed the role of succinoglycan Ridin octaose in eliciting the defense response of Solanum tuberosum. **RENJIE et al.** APPLIED MICROBIOLOGY AND BIOTECHNOLOGY. SPRINGER, vol. 105, 7439-7450